# EUROPEAN PATENT APPLICATION

(11) **EP 4 793 351 A1**
(43) Date of publication of application: **19.08.2026**
(21) Application number: 24876627.1
(22) Date of filing: 11.10.2024
(51) Int. Cl.: C12N 15/00, C12N 15/64, C12N 15/65, C12Q 1/68, G01N 33/542, G01N 33/68, C40B 30/00

(54) **METHOD FOR CONSTRUCTING VECTOR PAIR, AND HOST CELL AND HIGH-EXPRESSION CELL STRAIN**

(30) Priority: 12.10.2023 CN 202311324995
(71) Applicant: WuXi Biologics Ireland Limited, Dundalk, Co Louth A91 X56F (IE)
(72) Inventor: LIAN, Dujuan, Shanghai 200131 (CN); SHEN, Yang, Shanghai 200131 (CN); LIAO, Shanhui, Shanghai 200131 (CN); GAO, Rong, Shanghai 200131 (CN); LIU, Tongxin, Shanghai 200131 (CN); WU, Lingyan, Shanghai 200131 (CN); ZHANG, Yu, Shanghai 200131 (CN); ZHANG, Sheng, Shanghai 200131 (CN)
(74) Representative: Modiano, Gabriella Diana
(86) International application number: PCT/CN2024/124097
(87) International publication number: WO 2025/077816

(57) **Abstract**

Disclosed in the present application is a method for constructing a vector pair, and a host cell and a high-expression cell strain. The vector pair comprises a first vector and a second vector, the first vector comprises an expression sequence of a first fragment of a bleomycin resistance protein, the second vector comprises an expression sequence of a second fragment of the bleomycin resistance protein, and the first vector and/or the second vector further comprises an expression sequence of a target protein.

## Description

### TECHNICAL FIELD

The present application relates to the technical field of protein expression, and specifically pertains to a vector pair, a host cell, and a method for constructing a high-expression cell line by utilizing a split bleomycin resistance protein.

### BACKGROUND

In recent years, an increasing number of biopharmaceuticals, and especially macromolecular drugs, have been successfully applied in the treatment of various diseases, such as cancer, immune and metabolic diseases, and central nervous system diseases. With the growing clinical needs, there are higher requirements for the yield and quality of macromolecular drugs. Recombinant protein technology is widely used in the research and development and production processes of macromolecular drugs. Various expression systems, including bacteria, fungi, plant cells, insect cells, and mammalian cells, *etc.*, can be used to express different kinds of protein drugs. Among them, the mammalian expression system has an absolute advantage in the biopharmaceutical field due to its ability to produce proteins with human-like post-translational modifications. However, the construction of mammalian cell lines is very time-consuming and labor-intensive, and often encounters problems such as low protein expression levels, unsatisfactory quality, poor stability, *etc*. Constructing a high-expression stable mammalian cell line is crucial for reducing drug production costs and ensuring drug quality.

Selection markers such as antibiotics, fluorescent proteins and the like are widely used in the field of recombinant protein expression and gene editing for screening cell lines stably expressing specific genes or proteins. However, when multiple genes need to be introduced, it is often limited by the insufficient number of selection markers, especially in the terms of biopharmaceuticals, where the types of available selection markers are relatively limited, and mainly include antibiotic and metabolic enzyme selection markers such as DHFR and GS. As the developed molecules have more complex structures, such as bispecific antibodies, multispecific antibodies, etc., the simultaneous expression of multiple heterologous polypeptide chains is required. This may necessitate the use of multiple selection pressures, which inevitably negatively exerts adverse effects on cell growth, and the use of multiple selection resistances also increases production costs. Protein-fragment complementation (PCA) refers to a technique in which a protein is split into two polypeptide fragments, and the two polypeptide fragments are respectively fused to complementary protein segments, such as GCN4 leucine zipper sequences. Due to the formation of the leucine zipper, the two polypeptide fragments can reassemble into a functional protein. This technology is not only an important tool for studying protein folding, evolution, subunit function regulation, and protein-protein interactions but also provides an effective strategy for addressing limitations associated with selection markers. Pelletier and Levray *et al.* have demonstrated that two complementary fragments of murine dihydrofolate reductase (DHFR) protein can be reconstituted through parallel leucine zippers, thereby restoring, to a certain extent, the activity and function of the protein. In 2000, it was reported by Ghosh *et al.* that when two complementary GFP protein fragments were respectively fused to antiparallel leucine zipper structures, an active GFP protein could be reassembled at both molecular and cellular levels.

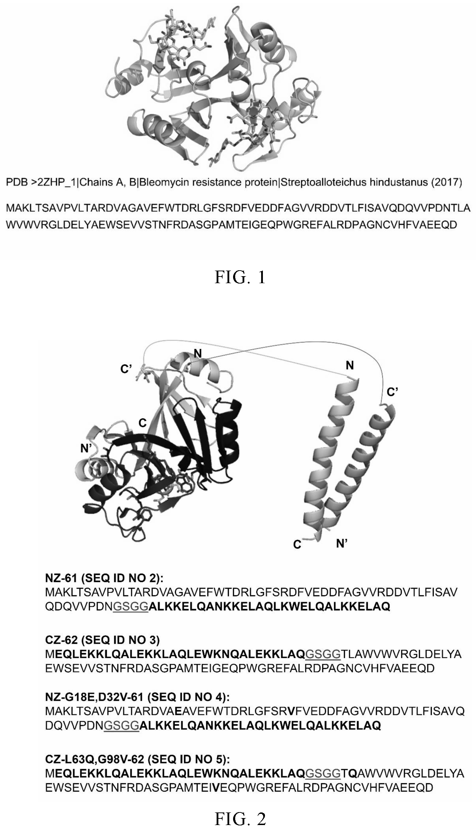

In mammalian cell expression systems, commonly used antibiotics mainly include geneticin (G418), Blasticidin, Hygromycin B, Puromycin, and Zeocin (bleomycin), and the corresponding resistance genes are NeoR, BsdR, HygR, PuroR, and BleoR, respectively. Studies have shown that the protein expression levels of cells screened with different types of antibiotics vary. Among them, bleomycin provides the best screening efficiency, resulting in cell lines with the highest protein expression and the smallest expression differences among clones. G418 and blasticidin result in cell lines with the lowest protein expression and the largest expression differences among clones, while puromycin and hygromycin fall intermediate between these two. Bleomycin has strong DNA cleavage activity and is capable of killing cell at concentrations ranging from 0.5 to 1000 µg/ml in conventional cell types. Cells expressing the bleomycin resistance gene can grow normally in culture medium containing bleomycin. The resistance protein encoded by the bleomycin resistance gene has a molecular weight of 14 kd, and its monomer forms a dimer and binds two bleomycin molecules (Figure 1), thereby blocking the cell-killing effect of bleomycin. However, to date, there has been no report on applying the split protein technology/protein-fragment complementation to the bleomycin resistance protein.

There is still a need in the art to construct vectors, host cells, and methods for applying the split protein technology/protein-fragment complementation to the bleomycin resistance protein, so as to achieve efficient screening of stable transfected cell lines using only one antibiotic pressure, and to construct high-expression monoclonal cell lines.

### SUMMARY OF INVENTION

To solve the above technical problems, one aspect of the present disclosure provides a vector pair comprising a first vector and a second vector, wherein the first vector comprises an expression sequence of a first fragment of a bleomycin resistance protein, the second vector comprises an expression sequence of a second fragment of the bleomycin resistance protein, and the first vector and/or the second vector further comprises an expression sequence of a target protein.

Another aspect of the present disclosure provides a host cell comprising a vector pair. The vector pair comprises a first vector and a second vector, wherein the first vector comprises an expression sequence of a first fragment of a bleomycin resistance protein, the second vector comprises an expression sequence of a second fragment of the bleomycin resistance protein, and the first vector and/or the second vector further comprises an expression sequence of a target protein.

Another aspect of the present disclosure provides a method for constructing a high-expression cell line, the method comprises:
a. obtaining a vector pair comprising a first vector and a second vector, wherein the first vector comprises an expression sequence of a first fragment of a bleomycin resistance protein, the second vector comprises an expression sequence of a second fragment of the bleomycin resistance protein, and the first vector and/or the second vector further comprises an expression sequence of a target protein;
b. transfecting the vector pair from step a into a host cell;
c. culturing the host cell from step b using a cell culture medium, wherein the cell culture medium comprises at least 50 µg/ml of bleomycin, preferably 50-500 µg/ml of bleomycin; and
d. sorting the screened/cultured host cell from step c to obtain the high-expression cell line.

### DESCRIPTION OF DRAWINGS

The present application is described in more detail below with reference to the accompanying drawings.
FIGURE 1 is a schematic diagram of the three-dimensional structure of the bleomycin resistance protein dimer and the amino acid sequence of the monomer (SEQ ID NO: 1).
FIGURE 2 is a schematic diagram of the three-dimensional structure and amino acid sequences of the bleomycin resistance protein split site N61|T62 and mutants (NZ-61 is SEQ ID NO: 2, CZ-62 is SEQ ID NO: 3, NZ-G18E,D32V-61 is SEQ ID NO: 4, CZ-L63Q,G98V-62 is SEQ ID NO: 5), wherein the red underline indicates the linker GSGG, and the bold sequence is the complementary antiparallel leucine zipper.

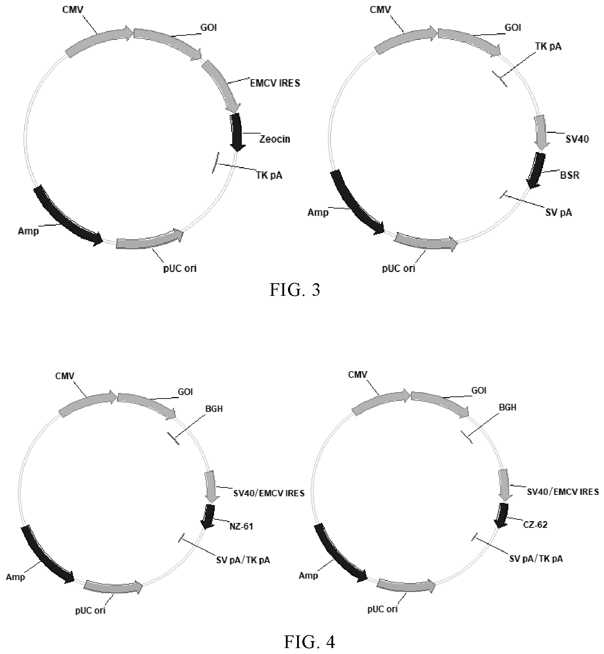

FIGURE 3 is a schematic diagram of the dual-pressure expression system vectors.
FIGURE 4 is a schematic diagram of the expression vectors for the N61|T62 split bleomycin resistance protein.

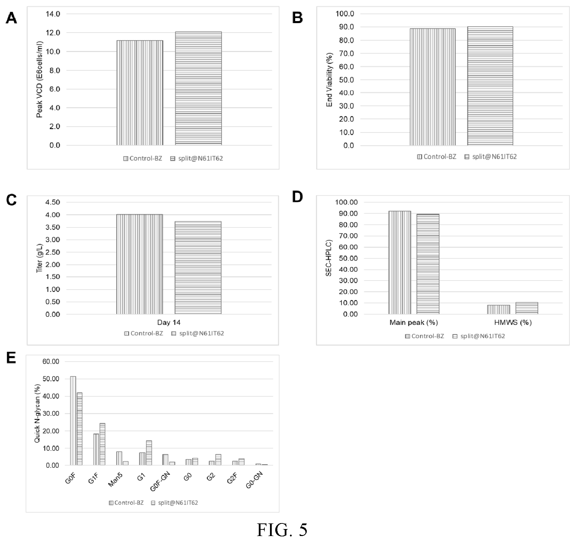

FIGURE 5 is a schematic diagram of the results of the N61|T62 split bleomycin resistance protein in monoclonal antibody expression screening, in which different groups of plasmids were transfected and selected under pressure. After recovery to normal viability, stable cell pools were subjected to fed-batch culture for 14 days; (A) peak viable cell density (VCD) during fed-batch culture; (B) cell viability at harvest on Day 14 of fed-batch culture; (C) protein expression titer on Day 14 during fed-batch culture; (D) size exclusion chromatography analysis (SEC-HPLC) of the harvested supernatant after one-step Protein A purification; and (E) glycan profile detection results of the one-step purified sample.

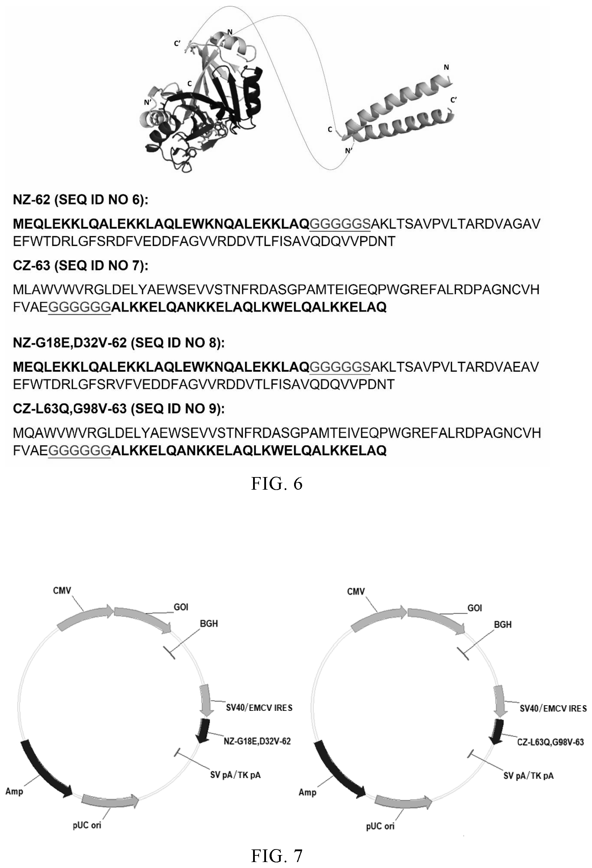

FIGURE 6 is a schematic diagram of the three-dimensional structure and amino acid sequences of the bleomycin resistance protein split site T62|L63 and mutants (NZ-61 is SEQ ID NO: 6, CZ-63 is SEQ ID NO: 7, NZ-G18E,D32V-62 is SEQ ID NO: 8, CZ-L63Q,G98V-63 is SEQ ID NO: 9).
FIGURE 7 is a schematic diagram of the expression vectors for the T62|L63 mutant split bleomycin resistance protein.

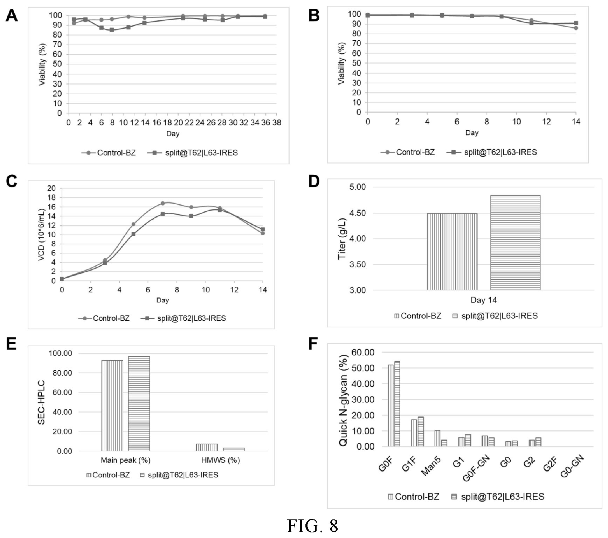

FIGURE 8 is a schematic diagram of the results of the T62|L63 mutant split bleomycin resistance protein in monoclonal antibody expression screening. The control group was transfected with HC-bleomycin resistance protein and LC-blasticidin resistance protein plasmids; and the splitT62|L63-IRES group was transfected with NZ-G18E,D32V-62-HC and CZ-L63Q,G98V-63-LC plasmids; (A) changes in cell pool viability over time after transfection; (B) viability changes during FB culture after cell pool recovery; (C) viable cell density (VCD) changes during FB culture of the cell pool; (D) monoclonal antibody expression level on Day 14 of FB culture; (E) SEC-HPLC results of one-step purified samples from the cell pool supernatant; and (F) glycan profile analysis results of the one-step purified samples.

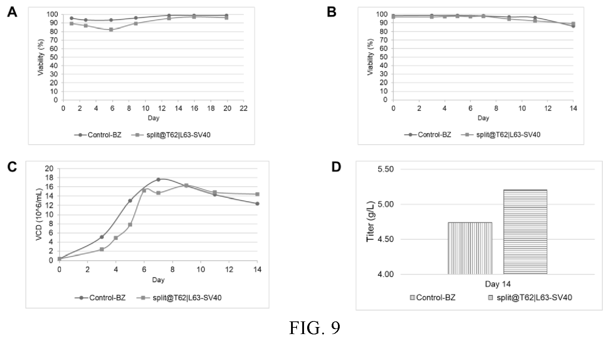

FIGURE 9 is a schematic diagram of the results of optimizing vector to improve the screening activity of the split protein; (A) viability changes during cell pool recovery after transfection; (B-C) viability (B) and VCD (C) changes during FB culture after cell pool recovery; and (D) monoclonal antibody expression level on Day 14 of FB culture of the cell pool.

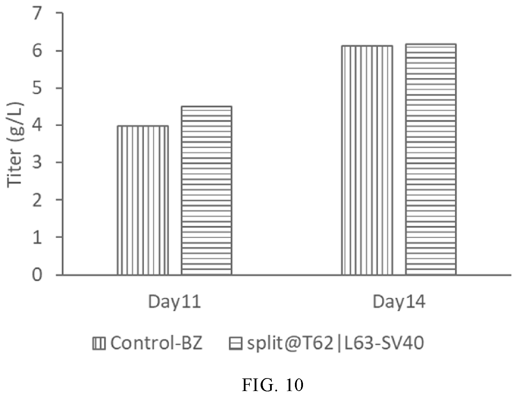

Figure 10 is a schematic diagram of the results of the T62|L63 mutant split bleomycin resistance protein in monoclonal antibody clone screening, in which single-cell clone screening was performed on stable transfected cell pools. The highest monoclonal antibody expression levels in the control group and the split protein group on Days 11 and 14 of fed-batch culture were shown, respectively.

### DETAILED DESCRIPTION

The present application relates to a vector pair comprising a first vector and a second vector. In the present application, the first and second vectors can be any expression vector known in the art and suitable for protein expression in host cells. In one embodiment of the present application, the first and second vectors include, but are not limited to, mammalian expression vectors (e.g., pcDNA3.3 and pOptiVEC), yeast cell expression vectors (e.g., pGAPZ), and insect cell expression vectors (e.g., pFastBac and pIB/V5-His). In one embodiment of the present application, the first vector comprises an expression sequence of a first fragment of a bleomycin resistance protein, and the second vector comprises an expression sequence of a second fragment of the bleomycin resistance protein. In one embodiment of the present application, the bleomycin resistance protein consists of the first fragment of the bleomycin resistance protein and the second fragment of the bleomycin resistance protein. In one embodiment of the present application, the amino acid sequence of the bleomycin resistance protein has at least 80%, 85%, 90%, 95%, or 100% homology with SEQ ID NO: 1. In one embodiment of the present application, the amino acid sequence of the bleomycin resistance protein is SEQ ID NO: 1. In one embodiment of the present application, the amino acid sequence of the first fragment of the bleomycin resistance protein has at least 80%, 85%, 90%, 95%, or 100% homology with SEQ ID NO: 2. In one embodiment of the present application, the amino acid sequence of the second fragment of the bleomycin resistance protein has at least 80%, 85%, 90%, 95%, or 100% homology with SEQ ID NO: 3. In one embodiment of the present application, the amino acid sequence of the first fragment of the bleomycin resistance protein is SEQ ID NO: 2. In one embodiment of the present application, the amino acid sequence of the second fragment of the bleomycin resistance protein is SEQ ID NO: 3. In one embodiment of the present application, the amino acid sequence of the first fragment of the bleomycin resistance protein has at least 80%, 85%, 90%, 95%, or 100% homology with SEQ ID NO: 6. In one embodiment of the present application, the amino acid sequence of the second fragment of the bleomycin resistance protein has at least 80%, 85%, 90%, 95%, or 100% homology with SEQ ID NO: 7. In one embodiment of the present application, the amino acid sequence of the first fragment of the bleomycin resistance protein is SEQ ID NO: 6. In one embodiment of the present application, the amino acid sequence of the second fragment of the bleomycin resistance protein is SEQ ID NO: 7. In one embodiment of the present application, the ratio of the first vector to the second vector is about 1:3 to 3:1.

In one embodiment of the present application, the first fragment of the bleomycin resistance protein and the second fragment of the bleomycin resistance protein comprise protein-binding motifs that are capable of binding with each other. In the present application, the protein-binding motifs are peptide fragments known in the art that can specifically bind to another protein-binding motif, and upon binding, can restore at least part of the activity, for example, restore full activity, of the full-length protein, to which other inactive protein fragments connected. In a preferred embodiment of the present application, the protein-binding motif comprises a leucine zipper. In one embodiment of the present application, the leucine zipper comprises an N-terminal antiparallel leucine zipper and a C-terminal antiparallel leucine zipper. In one embodiment of the present application, the first fragment of the bleomycin resistance protein comprises an N-terminal antiparallel leucine zipper. In one embodiment of the present application, the second fragment of the bleomycin resistance protein comprises a C-terminal antiparallel leucine zipper. In one embodiment of the present application, the amino acid sequence of the N-terminal antiparallel leucine zipper has at least 80%, 85%, 90%, 95%, or 100% homology with SEQ ID NO: 10. In one embodiment of the present application, the amino acid sequence of the N-terminal antiparallel leucine zipper is SEQ ID NO: 10. In one embodiment of the present application, the amino acid sequence of the C-terminal antiparallel leucine zipper has at least 80%, 85%, 90%, 95%, or 100% homology with SEQ ID NO: 11. In one embodiment of the present application, the amino acid sequence of the C-terminal antiparallel leucine zipper is SEQ ID NO: 11. In one embodiment of the present application, the first fragment of the bleomycin resistance protein and/or the second fragment of the bleomycin resistance protein further comprises a linker peptide sequence. In one embodiment of the present application, the linker peptide sequence comprises a flexible linker peptide sequence. In one embodiment of the present application, the flexible linker peptide sequence is selected from: GGGGS, GSGG, and GGGGGG. In one embodiment of the present application, the C-terminus of the first fragment of the bleomycin resistance protein comprises a protein-binding motif and a linker peptide sequence, for example, a flexible linker peptide sequence and an N-terminal antiparallel leucine zipper. In one embodiment of the present application, the N-terminus of the second fragment of the bleomycin resistance protein comprises a protein-binding motif and a linker peptide sequence, for example, a flexible linker peptide sequence and a C-terminal antiparallel leucine zipper.

In one embodiment of the present application, the first fragment and/or the second fragment of the bleomycin resistance protein comprises mutations. In one embodiment of the present application, the first fragment of the bleomycin resistance protein comprises one or two mutations selected from the group consisting of G18E and D32V. In one embodiment of the present application, the first fragment of the bleomycin resistance protein comprises the mutations G18E and D32V. In one embodiment of the present application, the second fragment of the bleomycin resistance protein comprises one or two mutations selected from the group consisting of L63Q and G98V. In one embodiment of the present application, the second fragment of the bleomycin resistance protein comprises the mutations L63Q and G98V. In one embodiment of the present application, the amino acid sequence of the first fragment of the bleomycin resistance protein has at least 80%, 85%, 90%, 95%, or 100% homology with SEQ ID NO: 4. In one embodiment of the present application, the amino acid sequence of the first fragment of the bleomycin resistance protein is SEQ ID NO: 4. In one embodiment of the present application, the amino acid sequence of the second fragment of the bleomycin resistance protein has at least 80%, 85%, 90%, 95%, or 100% homology with SEQ ID NO: 5. In one embodiment of the present application, the amino acid sequence of the second fragment of the bleomycin resistance protein is SEQ ID NO: 5. In one embodiment of the present application, the amino acid sequence of the first fragment of the bleomycin resistance protein has at least 80%, 85%, 90%, 95%, or 100% homology with SEQ ID NO: 8. In one embodiment of the present application, the amino acid sequence of the first fragment of the bleomycin resistance protein is SEQ ID NO: 8. In one embodiment of the present application, the amino acid sequence of the second fragment of the bleomycin resistance protein has at least 80%, 85%, 90%, 95%, or 100% homology with SEQ ID NO: 9. In one embodiment of the present application, the amino acid sequence of the second fragment of the bleomycin resistance protein is SEQ ID NO: 9.

In one embodiment of the present application, the first vector and/or the second vector further comprises an expression sequence of a target protein. In the present application, the target protein can be any protein suitable for expression in a host cell. In one embodiment of the present application, one of the first vector and the second vector comprises an expression sequence of the target protein. In one embodiment of the present application, the first vector comprises a first expression sequence of the target protein, and the second vector comprises a second expression sequence of the target protein. In one embodiment of the present application, the expression sequence of the target protein consists of the first expression sequence and the second expression sequence of the target protein. In one embodiment of the present application, the first expression sequence of the target protein expresses a first target protein, and the second expression sequence of the target protein expresses a second target protein. In one embodiment of the present application, a functional protein can be formed from the first target protein and the second target protein. In one embodiment of the present application, the target protein comprises a heterologous or homologous protein, for example, a monoclonal antibody, a bispecific antibody, a fusion protein, or a recombinant protein. In a preferred embodiment of the present application, the target protein comprises a monoclonal antibody, for example, adalimumab. In one embodiment of the present application, the first target protein and the second target protein are two parts of an antibody, respectively. In one embodiment of the present application, the first target protein is the heavy chain of an antibody, and the second target protein is the light chain of the antibody. In another embodiment of the present application, the first target protein is the light chain of an antibody, and the second target protein is the heavy chain of the antibody. In one embodiment of the present application, the first target protein is the heavy chain of adalimumab, and the second target protein is the light chain of adalimumab. In one embodiment of the present application, the amino acid sequence of the first target protein has at least 80%, 85%, 90%, 95%, or 100% homology with SEQ ID NO: 12. In one embodiment of the present application, the amino acid sequence of the second target protein has at least 80%, 85%, 90%, 95%, or 100% homology with SEQ ID NO: 13. In one embodiment of the present application, the amino acid sequence of the first target protein is SEQ ID NO: 12. In one embodiment of the present application, the amino acid sequence of the second target protein is SEQ ID NO: 13. In one embodiment of the present application, the first fragment and/or the second fragment of the bleomycin resistance protein is linked to the expression sequence of a target protein via a linker sequence. In one embodiment of the present application, the first fragment of the bleomycin resistance protein is linked to the first expression sequence of a target protein via a linker sequence. In one embodiment of the present application, the second fragment of the bleomycin resistance protein is linked to the second expression sequence of a target protein via a linker sequence. In one embodiment of the present application, the linker sequence comprises an internal ribosome entry site (IRES) or a 2A peptide sequence. In one embodiment of the present application, the linker sequence comprises an EMCV IRES sequence.

In one embodiment of the present application, the first vector and/or the second vector further comprise a promoter. In one embodiment of the present application, the promoter is independently selected from the group consisting of CMV, EF1A, CAG, CBA, CBh, and SV40. In a preferred embodiment of the present application, the promoter comprises SV40. In one embodiment of the present application, the first vector and/or the second vector further comprise a regulatory element. In one embodiment of the present application, the regulatory element is independently selected from the group consisting of a MAR/SAR sequence, a spacer sequence, a UCOE sequence, and a STAR sequence.

The present application also relates to a host cell comprising the vector pair according to any one of the preceding embodiments of the present application. In a preferred embodiment of the present application, the host cell comprises a eukaryotic cell. In a more preferred embodiment of the present application, the host cell comprises a mammalian cell. In one embodiment of the present application, the host cell is selected from the group consisting of a CHO-K1 cell, CHO-S, CHO-DXB11, CHO-DG44, NS0, SP2/0, and HEK293. In a preferred embodiment of the present application, the host cell comprises a CHO-K1 cell.

The present application also relates to a method for constructing a high-expression cell line. The method comprises a. obtaining a vector pair according to any one of the preceding embodiments of the present application.

The method for constructing the high-expression cell line further comprises b. introducing/transfecting the vector pair from step a into a host cell. In one embodiment of the present application, step b comprises calcium phosphate co-precipitation, cationic polymer method, liposome transfection, electroporation, microinjection, or gene gun. In a preferred embodiment of the present application, step b comprises electroporation.

The method for constructing a high-expression cell line further comprises c. culturing the host cell from step b using a cell culture medium. In one embodiment of the present application, the cell culture medium comprises at least 50 µg/ml of bleomycin. In a preferred embodiment of the present application, the cell culture medium comprises 50-500 µg/ml of bleomycin. In the present application, the cell culture medium can be any medium known in the art suitable for the growth of a host cell. In one embodiment of the present application, step c comprises culturing at 25-40°C. In one embodiment of the present application, step c comprises culturing at 30-38°C. In one embodiment of the present application, step c comprises culturing at 35-37°C. In one embodiment of the present application, step c lasts for 1-5 days. In one embodiment of the present application, step c lasts for 2-4 days.

The method for constructing a high-expression cell line further comprises d. sorting the cultured host cell from step c to obtain the high-expression cell line. In one embodiment of the present application, step d comprises limiting dilution or solid medium picking.

The technical solutions of the present application will be described clearly and completely below with reference to the accompanying drawings. It should be understood that the embodiments described herein are only a part of the embodiments of the present application, and are not intended to be exhaustive. Based on the embodiments disclosed herein, other embodiments, examples, and variations may be made by those skilled in the art without departing from the spirit and scope of the present application, and all such embodiments, examples and variations are intended to fall within the scope of the present application.

### EXAMPLES

The technical solutions of the present disclosure will be described in more detail below with reference to specific examples. The following examples are illustrative purposes only and are not intended to limit or restrict the scope of the present disclosure. Specific technical parameters, such as materials, steps, conditions, numerical values, or numerical ranges described in the following examples are merely exemplary, and are not intended to exhaustive or limiting.

In addition to the specific methods, devices, and materials used in the examples, those of ordinary skill in the art, in view of the present disclosure and the prior art, will recognize that other methods, devices, and materials in the prior art that are similar or equivalent to those described in the examples of the present disclosure may also be used to implement the present disclosure.

**Table 1. List of Experimental Equipment**

| Name | Supplier | Model |
|---|---|---|
| Biorad Electroporator | BIO-RAD | Gene Pulser 1652661 |
| Shaking table | Kuhner | SMX1503C |
| proA purification equipment | cytiva | AKTA Pure M150 |
| SEC-HPLC | Thermo Fisher | Vanquish |
| Electroporation cuvette | BIO-RAD | 0.4 cm electrode |
| 96-well plate | COSTAR | Flat Bottom with Lid |
| Cell viability analyzer | BECKMAN COULTER | Vi-CELL XR |

**Table 2. List of Experimental Reagents**

| Name | Grade | Manufacturer/Supplier | Lot. No |
|---|---|---|---|
| CHO-K1 host cell | NA | ATCC | CCL61 |
| DPBS solution | Research grade | cytiva | SH30028.02 |
| Pressure-free culture medium | Research grade | Gibco | 12490 |
| Production culture medium | Research grade | Hyclone | SH31037 |
| Feed 1 | Research grade | Hyclone | SH31026 |
| Feed 2 | Research grade | Hyclone | SH31027 |
| Sugar Feed | Research grade | Avantor | 1919 |
| Human IgG HTRF kit | Research grade | cisbio | 6FHIGPEH |
| Bleomycin | Research grade | Thermo | R25005 |
| Blasticidin | Research grade | Gibco | A11139-03 |

To illustrate the technical solutions of the present disclosure more clearly, further description will be made with reference to the examples.

### Example 1. Use of N61|T62 split bleomycin resistance protein in developing stable adalimumab-expressing monoclonal antibody cell pools

### 1. Expression vector construction

1.1 Based on the commercial vector pcDNA3.3, two parts of the bleomycin resistance protein were cloned into the plasmid via restriction enzymes BglII and AgeI to replace the original neomycin resistance protein. According to the three-dimensional structural characteristics of the bleomycin resistance protein, it was split into two parts at the site N61|T62. The N-terminal part was linked to the C-terminal antiparallel leucine zipper sequence (SEQ ID NO: 11) via GSGG (designated as NZ-61), and the amino acid sequence of which is shown in SEQ ID NO: 2. The N-terminal antiparallel leucine zipper sequence (SEQ ID NO: 10) was linked to the C-terminal part via GSGG (designated as CZ-62), and the amino acid sequence of which is shown in SEQ ID NO: 3. The resulting structure is shown in Figure 2. The monoclonal antibody HC and LC expression vectors were constructed according to the plasmid schematic shown in Figure 4. The monoclonal antibody HC sequence (SEQ ID NO: 12) was constructed upstream of the NZ-61 expression sequence, and similarly, the monoclonal antibody LC sequence (SEQ ID NO: 13) was constructed upstream of the CZ-62 expression sequence, with both linked via an EMCV IRES sequence.

1.2 The control expression vector is shown in Figure 3. The monoclonal antibody HC and the complete bleomycin resistance gene were linked via an EMCV IRES sequence. The LC sequence was placed upstream of the blasticidin resistance gene. The LC and resistance gene elements belonged to two independent reading frames. The expression of the blasticidin resistance gene was driven by SV40 promoter.

### 2. Cell transfection and cell pool screening

2.1 A suspension of 1E7 CHO-K1 host cells was taken, centrifuged at 290g for 5 minutes, and the supernatant was discarded. 300 ul DPBS solution was added to resuspend evenly. 10 ug of HC expression vector and 10 ug of LC expression vector were added in 250 ul DPBS solution and mixed evenly, and the plasmid solution was mixed thoroughly with the cell suspension and added to an electroporation cuvette. The cuvette was placed in a slot of the Biorad electroporator and electroporation was performed according to the corresponding program for the host cells. After transfection, the cell suspension was aspirated from the cuvette and transferred to 10 mL of pre-warmed pressure-free culture medium, and then cultured at 36.5°C, 225 rpm in a shaking table.

2.2 Twenty-four hours after transfection, an equal volume of pressure-containing culture medium (pressure-free culture medium containing antibiotics) was added for screening. In this example, the blasticidin concentration for the control group was 18 µg/mL, the bleomycin concentration was 800 µg/mL, and the bleomycin concentration for the split protein group was 400 µg/mL. After culturing for 3-4 days, the cells were passaged into pressure-containing medium for screening. Only cells expressing the target vector survived.

### 3. Fed-Batch culture and detection of cell pools

3.1 After continuous passaging for 3-4 weeks, and when the cell pool viability recovered to above 95%, the cell pool was inoculated into a production culture medium at a density of 4E5 cells/mL for fed-batch culture. The cell samples were taken on Days 3, 5, 7, 9, and 11 of culture to determine viable cell density and viability using the Vi-CELL XR cell viability analyzer. Feeding and glucose supplementation were performed accordingly. On Days 9 and 14 of culture, an appropriate amount of cells was taken and centrifuged. The supernatant was taken, and the monoclonal antibody expression titer was detected using the ProA-HPLC method.

3.2 On Day 14 of culture, the cells were centrifuged, and the supernatant was harvested. One-step ProA purification was performed, followed by a high aggregate content detection by SEC-HPLC and a glycan profile analysis by the Quick N-glycan (QNG) method.

### 4. Experimental results

The cell pool screened by the dual-pressure system required approximately 2 weeks for growth recovery, while the recovery of the cell pool under the single-pressure system of the split bleomycin resistance protein took longer, approximately 3-4 weeks, suggesting that the activity of the bleomycin resistance protein after intracellular recombination might be lower than the wild-type full-length protein. From Figures 5A and 5B, it can be seen that during FB culture, the peak VCD and the viability at harvest of the split protein group cell pool were comparable to the control group. Figure 5C indicates that there was no significant difference in monoclonal antibody expression titer on Days 9 and 14 between the split protein group and the control group. Furthermore, SEC-HPLC results showed that the high aggregate level of the one-step purified samples from the split protein group cell pool was not significantly different from the control group (Figure 5D), indicating that this N61|T62 split protein method can effectively allow the two protein parts to reassemble into an active protein within the cell and function for screening even at lower bleomycin concentrations. Additionally, QNG glycan profile results (Figure 5E) showed that the Man5 level of the monoclonal antibody in the split protein group was lower than the control group. A literature indicates that Man5 levels are related to the half-life of antibody clearance in blood (Goetze, Andrew M., et al. "High-mannose glycans on the Fc region of therapeutic IgG antibodies increase serum clearance in humans." Glycobiology 21.7 (2011): 949-959), and the reduced Man5 level suggests that monoclonal antibodies produced by the split protein system may have advantages in *in vivo* stability.

### Example 2. Use of T62|L63 mutant split bleomycin resistance protein in developing stable adalimumab-expressing cell pools

### 1. Expression vector construction

Since amino acids 61-63 of the bleomycin resistance protein are critical sites for protein-bleomycin binding, the results from Example 1 indicated that the activity of the protein split at the N61|T62 site was significantly lower than that of the wild-type full-length protein. This may be due to the introduction of an antiparallel leucine zipper structure at this critical site, which may affect the protein-bleomycin binding. Therefore, to avoid introducing a reverse leucine zipper at this site and to improve the stability of the protein-bleomycin binding, the protein was split into N-terminal and C-terminal parts at the T62/L63 site. The N-terminal reverse leucine zipper was linked to the N-terminal protein via a GGGGS linker (designated as NZ-62), and the amino acid sequence of which is shown in SEQ ID NO: 4 (see Figure 6). The C-terminal reverse leucine zipper was linked to the C-terminus of the C-terminal protein via GGGGGGG (designated as CZ-63), and the amino acid sequence of which is shown in SEQ ID NO: 5 (see Figure 6). To further enhance the thermal stability of the bleomycin resistance protein, glycine (G) at position 18 in the NZ-62 protein was mutated to glutamic acid (E), and aspartic acid (D) at position 32 was mutated to valine (V). This mutant protein was designated as NZ-G18E,D32-62, and the amino acid sequence of which is shown in SEQ ID NO: 6 (see Figure 6). Leucine (L) at position 63 in the CZ-63 protein was mutated to glutamine (Q), and glycine (G) at position 98 was mutated to valine (V). This mutant protein was designated as CZ-L63Q,G98V-63, and the amino acid sequence of which is shown in SEQ ID NO: 6 (see Figure 6).

The T62|L63 mutant split bleomycin resistance protein was used in monoclonal antibody HC and LC expression vectors. As shown in the plasmid schematic in Figure 7, the monoclonal antibody HC sequence (SEQ ID NO: 10) was constructed upstream of the NZ-G18E,D32-62 expression sequence. Similarly, the monoclonal antibody LC sequence (SEQ ID NO: 11) was constructed upstream of the CZ-L63Q,G98V-63 expression sequence, with both linked via an EMCV IRES sequence. More preferably, the HC or LC sequence and the NZ-G18E,D32-62 expression sequence or the CZ-L63Q,G98V-63 expression sequence were placed in independent reading frames, and their expression was driven by CMV and SV40 promoters, respectively. The HC sequence was constructed before the CZ-L63Q,G98V-63 expression sequence, and the LC sequence was constructed before the NZ-G18E,D32-62 expression sequence. The control dual-pressure expression vector was the same as in Example 1 (see Figure 3).

### 2. Cell transfection and cell pool screening

2.1 A suspension of 2E7 CHO-K1 host cells were taken, centrifuged at 290g for 5 minutes, and the supernatant was discarded. 300 ul of calcium- and magnesium-free DPBS solution was added and resuspended evenly. 20 ug of HC expression vector and 20 ug of LC expression vector were added in 250 ul DPBS solution and mixed evenly, and then the plasmid solution was mixed thoroughly with the cell suspension and added to an electroporation cuvette. The cuvette was placed in a slot of the Biorad electroporator and electroporation was performed according to the corresponding program for the host cells. After transfection, the cell suspension was aspirated from the cuvette and transferred to 12.5 mL of pre-warmed pressure-free culture medium, and then cultured at 36.5°C, 225 rpm in a shaking table.

2.2 Twenty-four hours after transfection, an equal volume of pressure-containing culture medium (pressure-free medium containing antibiotics) was added for screening. In this example, the blasticidin concentration for the control group was 18 µg/mL, the bleomycin concentration was 800 µg/mL, and the bleomycin concentration for the split protein group was 400 µg/mL. After culturing for 3-4 days, the cells were passaged into a pressure-containing culture medium for screening. Only cells expressing the target vector survived.

### 3. Fed-Batch culture and detection of cell pools

3.1 After continuous passaging and until the cell pool viability was recovered to above 95%, the cell pool was inoculated into a production medium at a density of 4E5 cells/mL for fed-batch culture. The cell samples were taken on Days 3, 5, 7, 9, and 11 of culture to detect viable cell density and viability. Feeding and glucose supplementation were performed accordingly. On Day 14 of culture, an appropriate amount of cells was taken and centrifuged. The supernatant was taken, and the monoclonal antibody expression titer was detected.

3.2 On Day 14 of culture, the cells were centrifuged, and the supernatant was harvested. One-step proA purification was performed, followed by SEC-HPLC for high aggregate content detection and Quick N-glycan for glycan profile analysis.

### 4. Experimental results

As shown in Figure 8A, the viability of the cell pool screened by the T62/L63 mutant split bleomycin resistance protein (hereinafter referred to as the T62|L63 mutant group) was lower than that of the control group after transfection. However, after 2-3 weeks, the viability recovered to above 95%, comparable to the control group. During FB culture, the viability changes of the T62|L63 mutant group were consistent with the control group (Figure 8B), and the VCD was slightly lower than the control group (Figure 8C), however, the monoclonal antibody titer on Day 14 was higher than that of the control group (Figure 8D), indicating that the T62|L63 mutant split bleomycin resistance protein can effectively screen for high-expression cells. SEC-HPLC results showed that the main peak purity of the T62|L63 mutant group was higher than that of the control group, while the aggregate purity was lower (Figure 8E). Glycan profile results showed that there is no significant difference in G0F, G1F, G0, G1, G2, and G0F-GN content compared to the control group, whereas the Man5 content was significantly lower than that of the control group (Figure 8F).

Immediately after expression vector optimization, the SV40 promoter was used to drive the expression of the split bleomycin resistance protein, the recovery time of the cell pool was significantly shortened, with cell viability recovering to above 95% after 2 weeks (Figure 9A). The growth during FB culture showed no significant difference compared to the control group (Figures 9B-C). The monoclonal antibody titer on Day 14 was about 10% higher than the control group (Figure 9D).

### Example 3. Use of T62|L63 mutant split bleomycin resistance protein in screening stable monoclonal antibody-expressing monoclonal cells

3.1 Single-cell printing technology was used for monoclonal sorting of the cell pools from the control group and the T62|L63 mutant split bleomycin resistance protein screening group in Example 2 (Figure 9A). A Single Cell Printer (SCP) was used to plate the suspension cells via an SCP chip at a density of one cell per well, with 4 96-well cell culture plates prepared per group. The culture medium used was a pressure-free culture medium containing additives. Images were taken on Days 1 and 7 after plating for monoclonal verification.

3.2 Around Day 12 after plating, the monoclonal antibody expression levels of the clones were detected using a human IgG HTRF kit (Cisbio). The clones with higher HTRF values, i.e., higher monoclonal antibody expression, were selected. These clones were transferred from their well positions on the 96-well plates to 24-well cell culture plates for static culture. The culture medium used was a pressure-containing medium supplemented with 200 mg/L bleomycin. After culturing in 24-well plates for 2-3 days, the clones were further transferred to cell culture tubes for shaking and passage culture. After passaging the selected clones for about 2 more weeks, they were inoculated at a density of 4E5 cells/mL into production culture medium for fed-batch culture. The cell samples were taken on Days 3, 5, 7, 9, and 11 of culture to detect viable cell density and viability, and feeding and glucose supplementation were performed accordingly. On Days 11 and 14 of culture, an appropriate amount of cells was taken and centrifuged. The supernatant was taken, and the monoclonal antibody expression titer was detected.

3.3 As shown in Figure 10, the highest titers of clones from the T62|L63 mutant split protein group on Days 11 and 14 were higher than those of the control group, suggesting that this strategy can effectively screen for high-expression clones.

In summary, compared with the prior art, the present disclosure has the following advantages.
1. The present disclosure, for the first time, applies the disclosed technology to the bleomycin resistance protein, enabling efficient screening of stably transfected cell lines and the construction of high-expression monoclonal cell lines using a single antibiotic pressure.
2. The present disclosure can reduce the use of antibiotics in the production process of macromolecular drugs, lower the concentration of bleomycin used, and due to the significantly higher expression levels compared to conventional dual-pressure screening systems, the production costs can be effectively reduced.
3. The molecules produced by the split bleomycin resistance protein screening system provided by the present disclosure have significantly lower high-mannose (Man5) levels than those produced by the dual-pressure screening system, which will be beneficial for improving the *in vivo* stability of the molecules.

The above are only specific examples of the present application and do not constitute any limitation on the scope of protection of the present application. For those skilled in the art, other variations or modifications in different forms may be made based on the above description. It is neither necessary nor possible to enumerate all implementation methods herein. All similar technical solutions formed by equivalent transformation or substitution fall within the scope of protection of the present application.

## Claims

1. A vector pair comprising a first vector and a second vector, wherein the first vector comprises an expression sequence of a first fragment of a bleomycin resistance protein, the second vector comprises an expression sequence of a second fragment of the bleomycin resistance protein, and the first vector and/or the second vector further comprises an expression sequence of a target protein.

2. The vector pair according to claim 1, wherein the amino acid sequence of the bleomycin resistance protein has at least 80%, 85%, 90%, 95%, or 100% homology with SEQ ID NO: 1.

3. The vector pair according to claim 1, wherein the first fragment of the bleomycin resistance protein and the second fragment of the bleomycin resistance protein comprise protein-binding motifs that are capable of binding with each other, preferably leucine zippers, for example, an N-terminal antiparallel leucine zipper and a C-terminal antiparallel leucine zipper, the amino acid sequence of the N-terminal antiparallel leucine zipper has at least 80%, 85%, 90%, 95%, or 100% homology with SEQ ID NO: 10, and the amino acid sequence of the C-terminal antiparallel leucine zipper has at least 80%, 85%, 90%, 95%, or 100% homology with SEQ ID NO: 11;
Optionally, the first fragment of the bleomycin resistance protein and/or the second fragment of the bleomycin resistance protein further comprises a linker peptide sequence, preferably a flexible linker peptide sequence, for example, the flexible linker peptide sequence is selected from GGGGS, GSGG, and GGGGGG; and
Optionally, the C-terminus of the first fragment of the bleomycin resistance protein comprises a protein-binding motif and a linker peptide sequence, for example, a flexible linker peptide sequence and an N-terminal antiparallel leucine zipper, and the N-terminus of the second fragment of the bleomycin resistance protein comprises a protein-binding motif and a linker peptide sequence, for example, a flexible linker peptide sequence and a C-terminal antiparallel leucine zipper.

4. The vector pair according to claim 1, wherein the amino acid sequence of the first fragment of the bleomycin resistance protein has at least 80%, 85%, 90%, 95%, or 100% homology with SEQ ID NO: 2; and the amino acid sequence of the second fragment of the bleomycin resistance protein has at least 80%, 85%, 90%, 95%, or 100% homology with SEQ ID NO: 3; or
The amino acid sequence of the first fragment of the bleomycin resistance protein has at least 80%, 85%, 90%, 95%, or 100% homology with SEQ ID NO: 6; and the amino acid sequence of the second fragment of the bleomycin resistance protein has at least 80%, 85%, 90%, 95%, or 100% homology with SEQ ID NO: 7.

5. The vector pair according to claim 4, wherein the first fragment of the bleomycin resistance protein comprises one or two mutations selected from the group consisting of G18E and D32V, and/or the second fragment of the bleomycin resistance protein comprises one or two mutations selected from the group consisting of L63Q and G98V.

6. The vector pair according to claim 5, wherein the amino acid sequence of the first fragment of the bleomycin resistance protein has at least 80%, 85%, 90%, 95%, or 100% homology with SEQ ID NO: 4; and/or the amino acid sequence of the second fragment of the bleomycin resistance protein has at least 80%, 85%, 90%, 95%, or 100% homology with SEQ ID NO: 5; or
The amino acid sequence of the first fragment of the bleomycin resistance protein has at least 80%, 85%, 90%, 95%, or 100% homology with SEQ ID NO: 8; and/or the amino acid sequence of the second fragment of the bleomycin resistance protein has at least 80%, 85%, 90%, 95%, or 100% homology with SEQ ID NO: 9.

7. The vector pair according to claim 1, wherein the target protein comprises a heterologous or homologous protein, for example, a monoclonal antibody, a bispecific antibody, a fusion protein, or a recombinant protein, preferably a monoclonal antibody, for example, adalimumab.

8. The vector pair according to claim 1, wherein the first vector and/or the second vector further comprises a promoter, the promoter is independently selected from the group consisting of CMV, EF1A, CAG, CBA, CBh, and SV40, preferably SV40; and/or the first vector and/or the second vector further comprises a regulatory element, the regulatory element is independently selected from the group consisting of a MAR/SAR sequence, a spacer sequence, a UCOE sequence, and a STAR sequence.

9. The vector pair according to claim 1, wherein the ratio of the first vector to the second vector is about 1:3 to 3:1.

10. A host cell comprising the vector pair according to any one of claims 1-9, wherein the host cell preferably comprises a eukaryotic cell, more preferably comprises a mammalian cell, even more preferably is selected from the group consisting of CHO-K1 cell, CHO-S, CHO-DXB11, CHO-DG44, NS0, SP2/0, and HEK293.

11. A method for constructing a high-expression cell line, the method comprises:
a. obtaining the vector pair according to any one of claims 1-9;
b. transfecting the vector pair from step a into a host cell;
c. culturing the host cell from step b using a cell culture medium, wherein the cell culture medium comprises at least 50 µg/ml of bleomycin, preferably 50-500 µg/ml; and
d. sorting the cultured host cell from step c to obtain the high-expression cell line.
